# FASCICULE DE BREVET EUROPEEN

(11) **EP 0 989 128 B1**
(45) Date de publication et mention de la délivrance du brevet: **21.03.2001**
(21) Numéro de dépôt: 99402147.5
(22) Date de dépôt: 30.08.1999
(51) Int. Cl.: C07D 401/06, A61K 7/13, C07D 401/14, C07D 209/14, C07D 209/42

(54) **4-Hydroxyindoles cationiques et leur utilisation pour la teinture d'oxydation des fibres kératiniques**
Kationische 4-Hydroxyindole und ihre Verwendung zum oxidativen Färben von Keratinfasern
4-Hydroxyindoles kationic and their use for oxidation dyeing of keratinous fibres

(30) Priorité: 21.09.1998 FR 9811751
(43) Date de publication de la demande: 29.03.2000
(73) Titulaire: L'OREAL, 75008 Paris (FR)
(72) Inventeur: Terranova, Eric, 92270 Bois Colombes (FR); Lagrange, Alain, 77700 Coupvray (FR); Fadli, Aziz, 77500 Chelles (FR)
(74) Mandataire: Goulard, Sophie

(56) Documents cités:
- EP-A- 0 428 441
- EP-A- 0 446 131
- EP-A- 0 850 638
- FR-A- 2 736 640

## Description

L'invention a pour objet de nouveaux dérivés du 4-hydroxyindole comportant au moins un groupement cationique Z, Z étant choisi parmi des chaînes aliphatiques quaternisées, des chaînes aliphatiques comportant au moins un cycle saturé quatemisé, et des chaînes aliphatiques comportant au moins un cycle insaturé quaternisé, leur utilisation à titre de coupleur pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements). Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

On connaît dans l'état de la technique et notamment dans les demandes EP-A-0446 131, FR-A-2736 640 et EP-A-0428 441 des dérivés de 4-hydroxyindoles utilisés comme coupleurs en teinture d'oxydation des cheveux. Or, la demanderesse vient maintenant de découvrir, de façon totalement inattendue et surprenante, qu'une nouvelle famille de dérivés du 4-hydroxyindole de formule (I) ci-après définie, comportant au moins un groupement cationique Z, Z étant choisi parmi des chaînes aliphatiques quatemisées, des chaînes aliphatiques comportant au moins un cycle saturé quaternisé, et des chaînes aliphatiques comportant au moins un cycle insaturé quatemisé, conviennent pour une utilisation comme coupleur pour la coloration d'oxydation, mais en outre qu'ils permettent d'obtenir des compositions tinctoriales conduisant à des colorations puissantes, dans une très large palette de nuances et présentant d'excellentes propriétés de résistance aux différents traitements que peuvent subir les fibres kératiniques. Enfin, ces composés s'avèrent être aisément synthétisables.

Ces découvertes sont à la base de la présente invention.

L'invention a donc pour premier objet des dérivés du 4-hydroxyindole de formule (I) suivante, et leurs sels d'addition avec un acide : 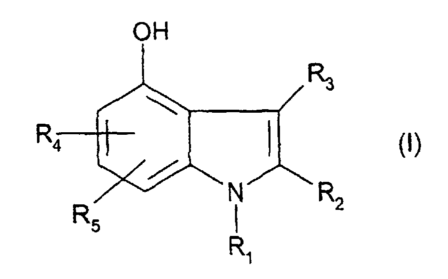 dans laquelle :
- R₁ représente un atome d'hydrogène ; un groupement Z ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle en C₁-C₄ ; un radical hydroxyalcoxy(C₁-C₄)alkyle en C₁-C₄ un radical aminoalkyle en C₁-C₄ ; un radical aminoalkyle en C₁-C₄ dont l'amine est mono ou disubstituée par un radical alkyle en C₁-C₄, par un radical acétyle, par un radical monohydroxyalkyle en C₁-C₄ ou par un radical polyhydroxyalkyle en C₂-C₄ ; un radical alkyle(C₁-C₄)thioalkyle en C₁-C₄, un radical monohydroxyalkyl(C₁-C₄)thioalkyle en C₁-C₄ ; un radical polyhydroxyalkyl(C2-C4)thioalkyle en C₁-C₄ ; un radical carboxyalkyle en C₁-C₄ ; un radical alcoxy(C₁-C₄)carbonylalkyle en C₁-C₄, un radical acétylaminoalkyle en C₁-C₄ ; un radical cyanoalkyle en C₁-C₄ ; un radical trifluoroalkyle en C₁-C₄ ; un radical halogénoalkyle en C₁-C₄ ; un radical phosphoalkyle en C₁-C₄ ou un radical sulfoalkyle en C₁-C₄ ;
- R₂ et R₃, identiques ou différents, représentent un atome d'hydrogène ou d'halogène ; un groupement Z ; un groupement -NH-Z ; un radical alkyle en C₁-C₄ ; un radical carboxyle ; un radical alcoxy(C₁-C₄)carbonyle ou un radical formyle ;
- R₄ et R₅, identiques ou différents, représentent un atome d'hydrogène ou d'halogène ; un groupement Z ; un groupement -NH-Z ; un radical alkyle en C₁-C₄ ; un radical alcoxy en C₁-C₄ ; un radical acétylamino ; un radical monohydroxyalkyle en C₁-C₅ ; un radical polyhydroxyalkyle en C₂-C₄ ; un radical alcoxy(C₁-C₄)alkyle en C₁-C₄ ; un radical thiophène ; un radical furane ; un radical phényle ; un radical aralkyle en C₁-C₄ ; un radical phényle ou aralkyle en C₁-C₄ substitué par un atome d'halogène, par un radical alkyle en C₁-C₄, par un radical trifluorométhyle, par un radical alcoxy en C₁-C₄, par un radical amino ou par un radical amino mono- ou disubstitué par un radical alkyle en C₁-C₄ ; un radical alkyl(C₁-C₄)aminoalkyle en C₁-C₄ ou un radical dialkyl(C₁-C₄)aminoalkyle en C₁-C₄ ;
- Z est choisi parmi les groupements cationiques insaturés de formules (II) et (III) suivantes, et les groupements cationiques saturés de formule (IV) suivante : 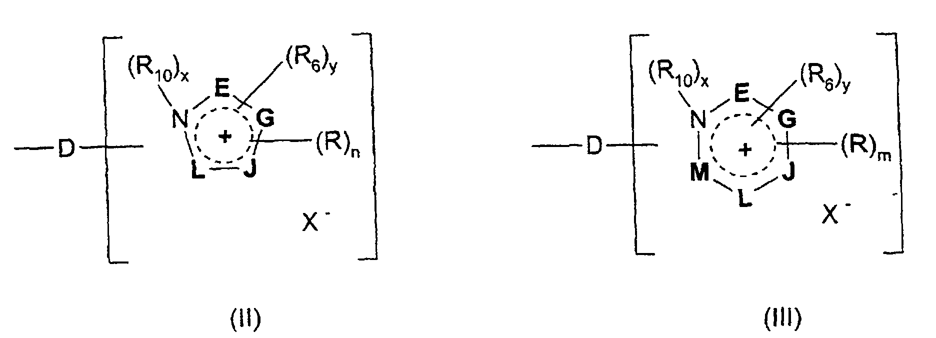 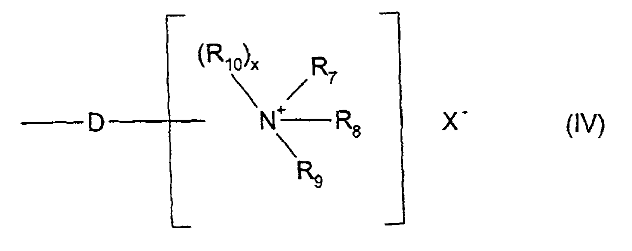 dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent un groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
   - R₆ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un groupement Z de formule (Il), (III) ou (IV) telles que définies ci-dessus ;
   - R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
      l'un des radicaux R₇, R₈ et R₉ peut également représenter un second groupement Z identique ou différent du premier groupement Z ;
   - R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆; un radical N-alkyl(C₁-C₆)sulfonamidoalkyle en C₁-C₆ ;
   - x et y sont des nombres entiers égaux à 0 ou 1 ; avec les conditions suivantes :
      - dans les groupements cationiques insaturés de formule (II) :
         - lorsque x = 0, le bras de liaison D est rattaché à l'atome d'azote,
         - lorsque x = 1, le bras de liaison D est rattaché à l'un des sommets E, G, J ou L,
         - y ne peut prendre la valeur 1 que :
            1) lorsque les sommets E, G, J et L représentent simultanément un atome de carbone, et que le radical R₆ est porté par l'atome d'azote du cycle insaturé ; ou bien
            2) lorsqu'au moins un des sommets E, G, J et L représente un atome d'azote sur lequel le radical R₆ est fixé ;
      - dans les groupements cationiques insaturés de formule (III) :
         - lorsque x = 0, le bras de liaison D est rattaché à l'atome d'azote,
         - lorsque x = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₆ est porté par l'atome d'azote du cycle insaturé ;
      - dans les groupements cationiques de formule (IV) :
         - lorsque x = 0, alors le bras de liaison est rattaché à l'atome d'azote portant les radicaux R₇ à R₉,
         - lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
   - X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
étant entendu que :
- le nombre de groupements cationiques Z de formule (II), (III) ou (IV) est au moins égal à 1.

Comme indiqué précédemment, les colorations obtenues avec la composition de teinture d'oxydation conforme à l'invention sont puissantes et permettent d'atteindre des nuances dans une très large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, et de la transpiration.

Dans la formule (I) ci-dessus les radicaux alkyle et alcoxy peuvent être linéaires ou ramifiés.

Parmi les cycles des groupements insaturés Z de formule (Il) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les composés de formule (1) ci-dessus, on peut notamment citer :
- le méthosulfate de 3-(4-hydroxy-1-méthyl-1H-indol-5-ylméthyl)1-méthyl-pyridinium ;
- le méthosulfate de 4-(4-hydroxy-1-méthyl-1H-indol-5-ylméthyl)-1-méthyl-pyridinium ;
- le diméthosulfate de 3-[3-(4-hydroxy-5-(1-méthylpyridinium)-4-ylméthyl-indol-1-yl)-propyl]-1-méthyl-imidazol-1-ium ;
- le méthosulfate de 4-(4-hydroxy-1-(2-hydroxyéthyl)-1H-indol-5-ylméthyl)-1-méthyl-pyridinium ;
- le diméthosulfate de 3-[3-(4-hydroxy-5-(1-méthylpyridinium)-5-ylméthyl-indol-1-yl)-propyl]-1-méthyl-imidazol-1-ium ;
- le diméthosulfate de 3-[4-hydroxy-5-(1-méthylpyridinium)-3-ylméthyl-indol-1-ylméthyl]-1-méthyl pyridinium ;
- le méthosulfate de 3-[3-(5-benzyl-4-hydroxy-indol-1-yl)-propyl]-1-méthyl-3H-imidazol-1-ium ;
- le méthosulfate de [2-(4-hydroxy-1H-indol-3-yl)-éthyl]-triméthyl-ammonium ;
- le méthosulfate de [2-(4-hydroxy-1-méthyl-1H-indol-3-yl)-éthyl]-triméthyl-ammonium ;
- le méthosulfate de (4-hydroxy-1-méthyl-1H-indol-3-ylméthyl)-triméthyl-ammonium ;
- le méthosulfate de (4-hydroxy-1H-indol-3-ylméthyl)-triméthyl-ammonium ;
- le méthosulfate de {3-[(4-hydroxy-1H-indol-2-carbonyl)-amino]-propyl}-triméthyl-ammonium ;
- le méthosulfate de {3-[(4-hydroxy-1-méthyl-1H-indol-2-carbonyl)-amino]-propyl}-triméthyl-ammonium;
- le méthosulfate de {3-[(4-hydroxy-5-méthyl-1H-indol-2-carbonyl)-amino]-propyl}-triméthyl-ammonium ;
- le méthosulfate de {3-((4-hydroxy-1,5-diméthyl-1H-indol-2-carbonyl)-amino]-propyl}-triméthyl-ammonium ;
- le méthosulfate de 3-{3-[(4-hydroxy-1H-indol-2-carbonyl)-amino]-propyl}-1-méthyl-3H-imidazol-1-ium ;
- le méthosulfate de 3-{3-[(4-hydroxy-1-méthyl-1H-indol-2-carbonyl)-amino]-propyl}-1 -méthyl-3H-imidazol-1-ium ;
- le méthosulfate de 3-{3-[(4-hydroxy-5-méthyl-1H-indol-2-carbonyl)-amino]-propyl}-1-méthyl-3H-imidazol-1-ium ;
- le méthosulfate de 3-{3-[(4-hydroxy-1,5-diméthyl-1H-indol-2-carbonyl)-amino]-propyl}-1 -méthyl-3H-imidazol-1-ium ;
- le monochlorure de {3-[(4-hydroxy-1H-indol-6-carbonyl)-amino]-propyl}-triméthyl-ammonium ;
- le monochlorure de {3-[(4-hydroxy-1-méthyl-1H-indol-6-carbonyl)-amino]-propyl}-triméthyl-ammonium ;
et leurs sels d'addition avec un acide.

Les composés de formule (I) conformes à l'invention peuvent être facilement obtenus, selon des méthodes bien connues de l'état de la technique telles que par exemple selon le procédé de préparation décrit dans la demande de brevet FR-A-2 736 640, suivi par une étape classique de quaternisation terminale.

Un autre objet de l'invention est l'utilisation des composés de formule (I) conformes à l'invention, à titre de coupleur, pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) conforme à l'invention.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Selon une forme de réalisation préférée de l'invention, la composition tinctoriale renferme en outre une ou plusieurs bases d'oxydation qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-((7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'a-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 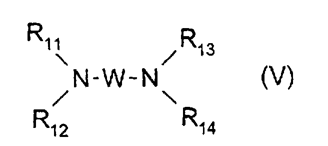 dans laquelle W est un reste propylène éventuellement substitué par un groupement hydroxyle ou un radical alkyle en C₁-C₆ ; R₁₁, R₁₂, R₁₃ et R₁₄, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE PREPARATION

### EXEMPLE DE PREPARATION 1 : Synthèse du méthosulfate de 3-(4-hydroxy-1-méthyl-1H-indol-5-ylméthyl)-1-méthyl-pyridinium

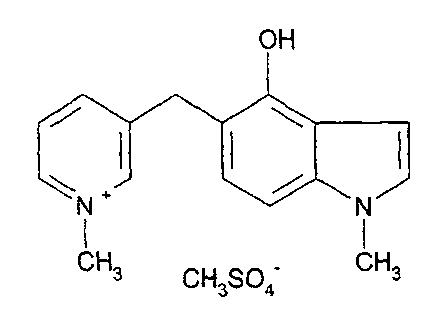

### a) Préparation du 1-méthyl-5-pyridin-3-ylméthyl-1H-indol-4-ol

Dans un réacteur de 1 litre, muni d'une agitation mécanique et surmonté d'un réfrigérant et d'un thermomètre, on a introduit 500 cc de méthyl-2-propanol-2, 75 g de 3-carboxaldéhyde pyridine et 100 g de 1-méthyl-1,5,6,7-tétrahydro-indol-4-one. Au bout de 10 minutes d'agitation, on a additionné en 15 minutes 157 g de tertiobutylate de potassium par petites portions. Après addition, on a maintenu la température à 75° C pendant 1 heure.
On a laissé revenir à température ambiante puis neutralisé (pH=6) avec de l'acide chlorhydrique à 20 %. On a versé sur 2 kg de glace-eau. Le précipité obtenu a été filtré, essoré et lavé à l'éther diisopropylique et à l'éther de pétrole. Il a été séché sur potasse sous vide à 30° C.
On a obtenu 120 g. de 1-méthyl-5-pyridin-3-ylméthyl-1H-indol-4-ol avec un rendement de 74%

### b) Préparation du méthosulfate de 3-(4-hydroxy-1-méthyl-1H-indol-5-ylméthyi)-1-méthyl-pyridinium

Dans un réacteur de 1 litre, muni d'une agitation mécanique et surmonté d'un réfrigérant et d'un thermomètre, on a introduit 95 g de 1-méthyl-5-pyridin-3-ylméthyl-1H-indol-4-ol dans 500 cc d'acétate d'éthyle et 53 g de diméthylsulfate. On a porté au reflux pendant 2 heures. On a laissé revenir à température ambiante et filtré le précipité. Le précipité a ensuite été lavé à l'acétate d'éthyle et à l'éther de pétrole, puis séché sur potasse sous vide à 30° C.
On a obtenu 140 g de méthosulfate de 3-(4-hydroxy-1-méthyl-1H-indol-5-ylméthyl)-1-méthyl-pyridinium avec un rendement de 96 %. Ce produit a ensuite été recristallisé dans le méthanol (2,5 cc/g) ; (rendement = 80 %).
L'analyse élémentaire calculée pour C₁₆H₁₇N₂O.CH₃O₄S (PM = 364,42 g) était :

| % | C | H | N | O | S |
|---|---|---|---|---|---|
| Calculé | 56.03 | 5.53 | 7.69 | 21.95 | 8.80 |
| Trouvé | 56.68 | 5.54 | 7.63 | 21.96 | 8.83 |

### EXEMPLES D'APPLICATION

### EXEMPLES 1 à 4 DE TEINTURE EN MILIEU ALCALIN

On a préparé les compositions tinctoriales conformes à l'invention suivantes (teneurs en grammes) :

| **EXEMPLE** | **1** | **2** | **3** | **4** |
|---|---|---|---|---|
| Méthosulfate de 3-(4-hydroxy-1-méthyl-1H-indol-5-ylméthyl)-1-méthyl-pyridinium (coupleur de formule (I)) | 1,093 | 1,093 | 1,093 | 1,093 |
| Para-aminophénol (base d'oxydation) | 0,327 | - | - | - |
| 2-β-acétylaminoéthoxy paraphénylènediamine, 2HCI (base d'oxydation) | - | 0,798 | - | - |
| 3-méthyl 4-amino phénol (base d'oxydation) | - | - | 0,369 | - |
| Paraphénylènediamine (base d'oxydation) | - | - | - | 0,327 |
| Support de teinture commun n°1 | (*) | (*) | (*) | (*) |
| Eau déminéralisée q.s.p. | 100 g | 100 g | 100 g | 100 g |

| | | | | |
|---|---|---|---|---|
| (*) Support de teinture commun n°1 : - Alcool éthylique à 96° 18 g - Métabisulfite de sodium en solution aqueuse à 35% 0,68 g - Sel pentasodique de l'acide diéthylènetriaminopentacétique 1,1 g - Ammoniaque à 20% 10,0 g | | | | |

Au moment de l'emploi, on a mélangé poids pour poids chacune des compositions tinctoriales ci-dessus avec une solution de peroxyde d'hydrogène à 20 volumes (6 % en poids) de pH 3.
Le mélange obtenu a été appliqué sur des mèches de cheveux gris permanentés à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Rouge irisé |
| **2** | 10 ± 0,2 | Bleu |
| **3** | 10 ± 0,2 | Irisé rouge |
| **4** | 10 ± 0,2 | Bleu violacé |

## Revendications

1. Composés de formule (I) suivante, et leurs sels d'addition avec un acide : 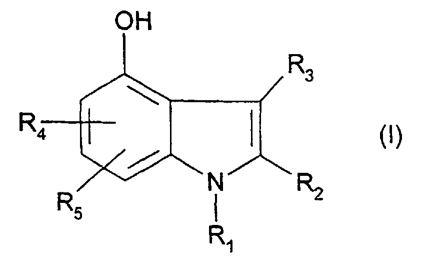 dans laquelle :
• R₁ représente un atome d'hydrogène ; un groupement Z ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle en C₁-C₄ ; un radical hydroxyalcoxy(C₁-C₄)alkyle en C₁-C₄ ; un radical aminoalkyle en C₁-C₄ ; un radical aminoalkyle en C₁-C₄ dont l'amine est mono ou disubstituée par un radical alkyle en C₁-C₄, par un radical acétyle, par un radical monohydroxyalkyle en C₁-C₄ ou par un radical polyhydroxyalkyle en C₂-C₄ ; un radical alkyle(C₁-C₄)thioalkyle en C₁-C₄, un radical monohydroxyalkyl(C₁-C₄)thioalkyle en C₁-C₄ ; un radical polyhydroxyalkyl(C₂-C₄)thioalkyle en C₁-C₄ ; un radical carboxyalkyle en C₁-C₄ ; un radical alcoxy(C₁-C₄)carbonylalkyle en C₁-C₄, un radical acétylaminoalkyle en C₁-C₄ ; un radical cyanoalkyle en C₁-C₄ ; un radical trifluoroalkyle en C₁-C₄ ; un radical halogénoalkyle en C₁-C₄ ; un radical phosphoalkyle en C₁-C₄ ou un radical sulfoalkyle en C₁-C₄ ;
• R₂ et R₃, identiques ou différents, représentent un atome d'hydrogène ou d'halogène ; un groupement Z ; un groupement -NH-Z ; un radical alkyle en C₁-C₄ ; un radical carboxyle ; un radical alcoxy(C₁-C₄)carbonyle ou un radical formyle ;
• R₄ et R₅, identiques ou différents, représentent un atome d'hydrogène ou d'halogène ; un groupement Z ; un groupement -NH-Z ; un radical alkyle en C₁-C₄ ; un radical alcoxy en C₁-C₄ ; un radical acétylamino ; un radical monohydroxyalkyle en C₁-C₅ ; un radical polyhydroxyalkyle en C₂-C₄ ; un radical alcoxy(C₁-C₄)alkyle en C₁-C₄ ; un radical thiophène ; un radical furane ; un radical phényle ; un radical aralkyle en C₁-C₄ ; un radical phényle ou aralkyle en C₁-C₄ substitué par un atome d'halogène, par un radical alkyle en C₁-C₄, par un radical trifluorométhyle, par un radical alcoxy en C₁-C₄, par un radical amino ou par un radical amino mono- ou disubstitué par un radical alkyle en C₁-C₄ ; un radical alkyl(C₁-C₄)aminoalkyle en C₁-C₄ ou un radical dialkyl(C₁-C₄)aminoalkyle en C₁-C₄ ;
• Z est choisi parmi les groupements cationiques insaturés de formules (Il) et (III) suivantes, et les groupements cationiques saturés de formule (IV) suivante : 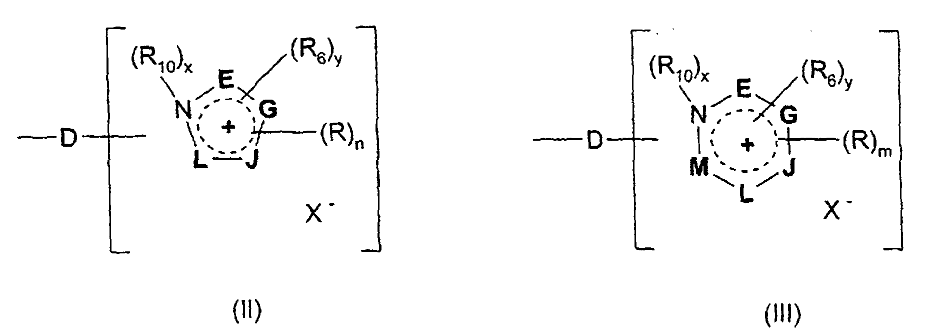 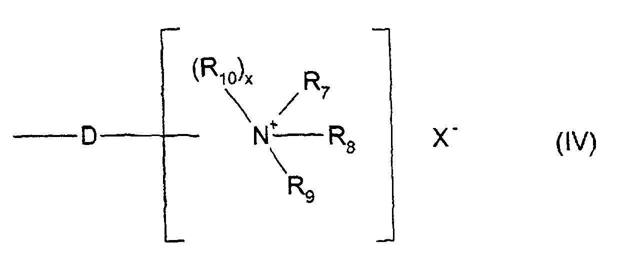 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₆ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un groupement Z de formule (Il), (III) ou (IV) telles que définies ci-dessus ;
• R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₇, R₈ et R₉ peut également représenter un second groupement Z identique ou différent du premier groupement Z ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆; un radical N-alkyl(C₁-C₆)sulfonamidoalkyle en C₁-C₆ ;
• x et y sont des nombres entiers égaux à 0 ou 1 ; avec les conditions suivantes :
- dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₆ est porté par l'atome d'azote du cycle insaturé ; ou bien
2) lorsqu'au moins un des sommets E, G, J et L représente un atome d'azote sur lequel le radical R₆ est fixé ;
- dans les groupements cationiques insaturés de formule (III):
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₆ est porté par l'atome d'azote du cycle insaturé. ;
- dans les groupements cationiques de formule (IV) :
- lorsque x = 0, alors le bras de liaison est rattaché à l'atome d'azote portant les radicaux R₇ à R₉,
- lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X⁻ représente un anion monovalent ou divalent ;
étant entendu que :
- le nombre de groupements cationiques Z de formule (II) ou (III) ou (IV) est au moins égal à 1.

2. Composés selon la revendication 1, caractérisés par le fait que les cycles des groupements insaturés Z de formule (II) sont choisis parmi les cycles pyrrolique, imidazolique, pyrazolique, oxazolique, thiazolique et triazolique.

3. Composés selon la revendication 1, caractérisés par le fait que les cycles des groupements insaturés Z de formule (III) sont choisis parmi les cycles pyridinique, pyrimidinique, pyrazinique, oxazinique et triazinique.

4. Composés selon l'une quelconque des revendications précédentes, caractérisés par le fait que deux des radicaux R₇, R₈ et R₉ forment un cycle pyrrolidinique, un cycle pipéridinique, un cycle pipérazinique ou un cycle morpholinique, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

5. Composés selon l'une quelconque des revendications précédentes, caractérisés par le fait que X⁻ est choisi parmi un atome d'halogène, un hydroxyde, un hydrogènesulfate, ou un alkyl(C₁-C₆)sulfate.

6. Composés selon l'une quelconque des revendications précédentes, caractérisés par le fait qu'ils sont choisis parmi :
- le méthosulfate de 3-(4-hydroxy-1-méthyl-1H-indol-5-ylméthyl)-1-méthylpyridinium ;
- le méthosulfate de 4-(4-hydroxy-1-méthyl-1H-indol-5-ylméthyl)-1-méthylpyridinium ;
- le diméthosulfate de 3-[3-(4-hydroxy-5-(1-méthylpyridinium)-4-ylméthyl-indol-1-yl)-propyl]-1-méthyl-imidazol-1-ium ;
- le méthosulfate de 4-(4-hydroxy-1-(2-hydroxyéthyl)-1H-indol-5-ylméthyl)-1-méthyl-pyridinium ;
- le diméthosulfate de 3-[3-(4-hydroxy-5-(1-méthylpyridinium)-5-ylméthyl-indol-1-yl)-propyl]-1-méthyl-imidazol-1-ium ;
- le diméthosulfate de 3-[4-hydroxy-5-(1-méthylpyridinium)-3-ylméthyl-indol-1-ylméthyl]-1-méthyl pyridinium ;
- le méthosulfate de 3-[3-(5-benzyl-4-hydroxy-indol-1-yl)-propyl]-1-méthyl-3H-imidazol-1-ium ;
- le méthosulfate de [2-(4-hydroxy-1H-indol-3-yl)-éthyl]-triméthyl-ammonium ;
- le méthosulfate de [2-(4-hydroxy-1-méthyl-1H-indol-3-yl)-éthyl]-triméthyl-ammonium ;
- le méthosulfate de (4-hydroxy-1-méthyl-1H-indol-3-ylméthyl)-triméthyl-ammonium ;
- le méthosulfate de (4-hydroxy-1H-indol-3-ylméthyl)-triméthyl-ammonium ;
- le méthosulfate de {3-[(4-hydroxy-1H-indol-2-carbonyl)-amino]-propyl}-triméthyl-ammonium ;
- le méthosulfate de {3-[(4-hydroxy-1-méthyl-1H-indol-2-carbonyl)-amino]-propyl}-triméthyl-ammonium ;
- le méthosulfate de {3-[(4-hydroxy-5-méthyl-1H-indol-2-carbonyl)-amino]-propyl}-triméthyl-ammonium;
- le méthosulfate de {3-[(4-hydroxy-1,5-diméthyl-1H-indol-2-carbonyl)-amino]-propyl}-triméthyl-ammonium ;
- le méthosulfate de 3-{3-[(4-hydroxy-1H-indol-2-carbonyl)-amino]-propyl}-1-méthyl-3H-imidazol-1-ium ;
- le méthosulfate de 3-{3-[(4-hydroxy-1-méthyl-1H-indol-2-carbonyl)-amino]-propyl}-1-méthyl-3H-imidazol-1-ium ;
- le méthosulfate de 3-{3-[(4-hydroxy-5-méthyl-1H-indol-2-carbonyl)-amino]-propyl}-1-méthyl-3H-imidazol-1-ium ;
- le méthosulfate de 3-{3-[(4-hydroxy-1,5-diméthyl-1H-indol-2-carbonyl)-amino]-propyl}-1-méthyl-3H-imidazol-1-ium ;
- le monochlorure de {3-[(4-hydroxy-1H-indol-6-carbonyl)-amino]-propyl}-triméthyl-ammonium ;
- le monochlorure de (3-[(4-hydroxy-1-méthyl-1H-indol-6-carbonyl)-amino]-propyl}-triméthyl-ammonium;
et leurs sels d'addition avec un acide.

7. Composés selon l'une quelconque des revendications précédentes, caractérisés par le fait que les sels d'addition avec un acide sont choisis parmi les chlorhydrates, les bromhydrates, les sulfates, les citrates, les succinates, les tartrates, les lactates et les acétates.

8. Utilisation des composés de formule (I) tels que définis à l'une quelconque des revendications 1 à 7, à titre de coupleur pour la teinture d'oxydation des fibres kératiniques, et en particulier des fibres kératiniques humaines telles que les cheveux.

9. Composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) tel que défini à l'une quelconque des revendications 1 à 7.

10. Composition selon la revendication 9, caractérisée par le fait que le ou les composés de formule (I) représentent de 0,0005 à 12 % en poids du poids total de la composition tinctoriale.

11. Composition selon la revendication 9, caractérisée par le fait que le ou les composés de formule (I) représentent de 0,005 à 6 % en poids du poids total de la composition tinctoriale.

12. Composition selon l'une quelconque des revendications 9 à 11, caractérisée par le fait qu'elle renferme une ou plusieurs bases d'oxydation choisies parmi les paraphénylènediamines, les bis-phénylalkylènediamines, les para-aminophénols, les ortho-aminophénols et les bases hétérocycliques.

13. Composition selon la revendication 12, caractérisée par le fait que les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl )amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

14. Composition selon la revendication 12, caractérisée par le fait que les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

15. Composition selon la revendication 12, caractérisée par le fait que les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

16. Composition selon la revendication 12, caractérisée par le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

17. Composition selon la revendication 12, caractérisée par le fait que les bases hétérocycliques sont choisies parmi les dérivés pyridiniques, les dérivés pyrimidiniques et les dérivés pyrazoliques.

18. Composition selon l'une quelconque des revendications 12 à 17, caractérisée par le fait que la ou les bases d'oxydation représentent de 0,0005 à 12 % en poids du poids total de la composition tinctoriale.

19. Composition selon la revendication 18, caractérisée par le fait que la ou les bases d'oxydation représentent de 0,005 à 6 % en poids du poids total de la composition tinctoriale.

20. Composition selon l'une quelconque des revendications 9 à 19, caractérisée par le fait qu'elle renferme un ou plusieurs coupleurs choisis parmi les métaphénylènediamines, les méta-aminophénols, les métadiphénols et les coupleurs hétérocycliques.

21. Composition selon la revendication 20, caractérisée par le fait que les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

22. Composition selon la revendication 20 ou 21, caractérisée par le fait que le ou les coupleurs représentent de 0,0001 à 10 % en poids environ du poids total de la composition tinctoriale.

23. Composition selon la revendication 22, caractérisée par le fait que le ou les coupleurs représentent de 0,005 à 5 % en poids environ du poids total de la composition tinctoriale.

24. Composition selon l'une quelconque des revendications 9 à 23, caractérisée par le fait que les sels d'addition avec un acide sont choisis parmi les chlorhydrates, les bromhydrates, les sulfates, les citrates, les succinates, les tartrates, les lactates et les acétates.

25. Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 9 à 24, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

26. Procédé selon la revendication 25, caractérisé par le fait que l'agent oxydant est choisi parmi le peroxyde d'hydrogène, le peroxyde d'urée, les bromates de métaux alcalins, les persels tels que les perborates et persulfates, et les enzymes telles que les peroxydases, les laccases et les oxydo-réductases à 2 électrons.

27. Dispositif à plusieurs compartiments, ou "kit" de teinture à plusieurs compartiments, dont un premier compartiment renferme une composition tinctoriale telle que définie à l'une quelconque des revendications 9 à 24 et un second compartiment renferme une composition oxydante.

## Claims

1. Compounds of formula (I) below and their addition salts with an acid: 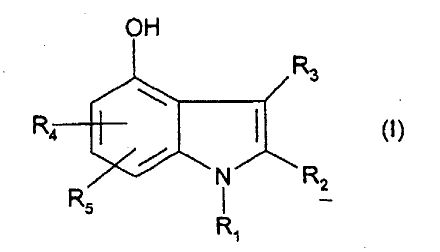 in which:
• R₁ represents a hydrogen atom; a group Z; a C₁-C₄ alkyl radical; a C₁-C₄ monohydroxyalkyl radical; a C₂-C₄ polyhydroxyalkyl radical; a (C₁-C₄ alkoxy)-C₁-C₄ alkyl radical; a hydroxy(C₁-C₄ alkoxy)-C₁-C₄ alkyl radical; a C₁-C₄ aminoalkyl radical; a C₁-C₄ aminoalkyl radical whose amine is mono- or disubstituted by a C₁-C₄ alkyl radical, by an acetyl radical, by a C₁-C₄ monohydroxyalkyl radical or by a C₂-C₄ polyhydroxyalkyl radical; a (C₁-C₄ alkyl)-C₁-C₄ thioalkyl radical, a monohydroxy(C₁-C₄ alkyl)-C₁-C₄ thioalkyl radical; a polyhydroxy(C₂-C₄ alkyl)-C₁-C₄ thioalkyl radical; a C₁-C₄ carboxyalkyl radical; a (C₁-C₄ alkoxy)-C₁-C₄ carbonylalkyl radical or a C₁-C₄ acetylaminoalkyl radical; a C₁-C₄ cyanoalkyl radical; a C₁-C₄ trifluoroalkyl radical; a C₁-C₄ haloalkyl radical; a C₁-C₄ phosphoalkyl radical, or a C₁-C₄ sulphoalkyl radical;
• R₂ and R₃, which are identical or different, represent a hydrogen or halogen atom; a group Z; a group -NH-Z; a C₁-C₄ alkyl radical; a carboxyl radical; a (C₁-C₄ alkoxy)carbonyl radical or a formyl radical;
• R₄ and R₅, which are identical or different, represent a hydrogen or halogen atom; a group Z; a group -NH-Z; a C₁-C₄ alkyl radical; a C₁-C₄ alkoxy radical; an acetylamino radical; a C₁-C₅ monohydroxyalkyl radical; a C₂-C₄ polyhydroxyalkyl radical; a (C₁-C₄ alkoxy)-C₁-C₄ alkyl radical; a thiophene radical; a furan radical; a phenyl radical; a C₁-C₄ aralkyl radical; a phenyl radical or C₁-C₄ aralkyl radical, each substituted by a halogen atom, by a C₁-C₄ alkyl radical, by a trifluoromethyl radical, by a C₁-C₄ alkoxy radical, by an amino radical or by an amino radical which is mono- or disubstituted by a C₁-C₄ alkyl radical; a (C₁-C₄ alkyl)-C₁-C₄ aminoalkyl radical or a di(C₁-C₄ alkyl)-C₁-C₄ aminoalkyl radical;
• Z is selected from the unsaturated cationic groups of formulae (II) and (III) below and the saturated cationic groups of formula (IV) below: 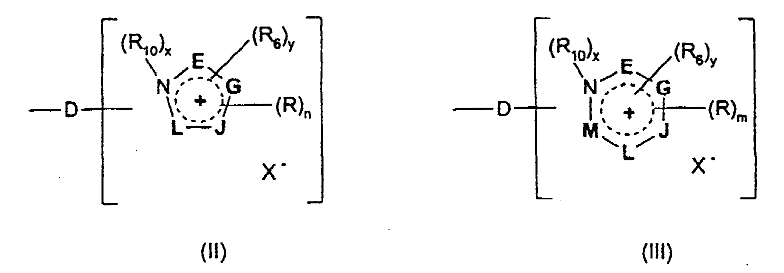 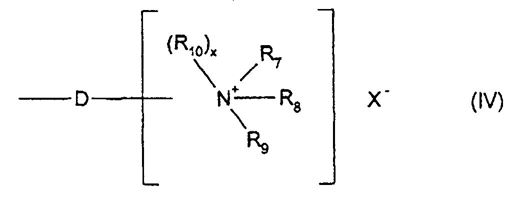 in which:
• D is a linker which represents an alkyl chain containing preferably 1 to 14 carbon atoms, which is linear or branched and can be interrupted by one or more heteroatoms such as oxygen, sulphur or nitrogen atoms and can be substituted by one or more hydroxyl or C₁-C₆ alkoxy radicals and can carry one or more ketone functional groups;
• the ring members E, G, J, L and M, which are identical or different, represent a carbon, oxygen, sulphur or nitrogen atom;
• n is an integer of between 0 and 4 inclusive;
• m is an integer of between 0 and 5 inclusive;
• the radicals R, which are identical or different, represent a group Z, a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a tri(C₁-C₆ alkyl)silyl-C₁-C₆ alkyl radical, an amido radical, an aldehydo radical, a carboxyl radical, a C₁-C₆ alkylcarbonyl radical, a thio radical, a C₁-C₆ thioalkyl radical, a (C₁-C₆ alkyl)thio radical, an amino radical, an amino radical protected by a (C₁-C₆ alkyl)carbonyl, carbamyl or (C₁-C₆ alkyl)sulphonyl radical; a group NHR'' or NR''R''' in which R'' and R"', which are identical or different, represent a C₁-C₆ alkyl radical, a C₁-C₆ monohydroxyalkyl radical or a C₂-C₆ polyhydroxyalkyl radical;
• R₆ represents a C₁-C₆ alkyl radical, a C₁-C₆ monohydroxyalkyl radical, a C₂-C₆ polyhydroxyalkyl radical, a C₁-C₆ cyanoalkyl radical, a tri(C₁-C₆ alkyl)silyl-C₁-C₆ alkyl radical, a (C₁-C₆ alkoxy)-C₁-C₆ alkyl radical, a C₁-C₆ carbamylalkyl radical, a (C₁-C₆ alkyl)-C₁-C₆ carboxyalkyl radical, a benzyl radical or a group Z of formula (II), (III) or (IV) as defined above;
• R₇, R₈ and R₉, which are identical or different, represent a C₁-C₆ alkyl radical, a C₁-C₆ monohydroxyalkyl radical, a C₂-C₆ polyhydroxyalkyl radical, a (C₁-C₆ alkoxy)-C₁-C₆ alkyl radical, a C₁-C₆ cyanoalkyl radical, an aryl radical, a benzyl radical, a C₁-C₆ amidoalkyl radical, a tri(C₁-C₆ alkyl)silyl-C₁-C₆ alkyl radical or a C₁-C₆ aminoalkyl radical whose amine is protected by a (C₁-C₆ alkyl)carbonyl, carbamyl or (C₁-C₆ alkyl)-sulphonyl radical; where two of the radicals R₇, R₈ and R₉ may also form, together with the nitrogen atom to which they are attached, a 5- or 6-membered saturated ring containing carbon or containing one or more heteroatoms, it being possible for the said ring to be unsubstituted or substituted by a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a tri(C₁-C₆ alkyl)silyl-C₁-C₆ alkyl radical, an amido radical, an aldehydo radical, a carboxyl radical, a C₁-c₆ ketoalkyl radical, a thio radical, a C₁-C₆ thioalkyl radical, a (C₁-C₆ alkyl)thio radical, an amino radical, or an amino radical protected by a (C₁-C₆ alkyl)carbonyl, carbamyl or (C₁-C₆ alkyl)sulphonyl radical; one of the radicals R₇, R₈ and R₉ may also represent a second group Z which is identical to or different from the first group z;
• R₁₀ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical or a C₁-C₆ aminoalkyl radical whose amine is protected by a (C₁-C₆ alkyl)carbonyl, carbamyl or (C₁-C₆ alkyl)sulphonyl radical; a C₁-C₆ carboxyalkyl radical; a C₁-C₆ cyanoalkyl radical; a C₁-C₆ carbamylalkyl radical; a C₁-C₆ trifluoroalkyl radical; a tri(C₁-C₆ alkyl)silyl-C₁-C₆ alkyl radical; a C₁-C₆ sulphonamidoalkyl radical; a (C₁-C₆-alkyl)carboxy-C₁-C₆ alkyl radical; a (C₁-C₆ alkyl)sulphinyl-C₁-C₆ alkyl radical; a (C₁-C₆ alkyl)sulphonyl-C₁-C₆ alkyl radical; a (C₁-C₆ alkyl)keto-C₁-C₆ alkyl radical; an N-(C₁-C₆ alkyl)carbamyl-C₁-C₆ alkyl radical; or an N-(C₁-C₆ alkyl)sulphonamido-C₁-C₆ alkyl radical;
• x and y are integers equal to 0 or 1; with the following conditions:
- in the unsaturated cationic groups of formula (II):
- when x = 0, the linker D is attached to the nitrogen atom,
- when x = 1, the linker D is attached to one of the ring members E, G, J or L,
- y can adopt the value 1 only:
1) when the ring members E, G, J and L represent simultaneously a carbon atom and when the radical R₆ is carried by the nitrogen atom of the unsaturated ring; or else
2) when at least one of the ring members E, G, J and L represents a nitrogen atom to which the radical R₆ is attached;
- in the unsaturated cationic groups of formula (III):
- when x = 0, the linker D is attached to the nitrogen atom,
- when x = 1, the linker D is attached to one of the ring members E, G, J, L or M,
- y can adopt the value 1 only when at least one of the ring members E, G, J, L and M represents a divalent atom and when the radical R₆ is carried by the nitrogen atom of the unsaturated ring;
- in the cationic groups of formula (IV):
- when x = 0, the linker is attached to the nitrogen atom which carries the radicals R₇ to R₉,
- when x = 1, two of the radicals R₇ to R₉ form, conjointly with the nitrogen atom to which they are attached, a 5- or 6-membered saturated ring as defined above, and the linker D is carried by a carbon atom of the said saturated ring;
• X⁻ represents a monovalent or divalent anion;
with the proviso that:
- the number of cationic groups Z of formula (II), (III) or (IV) is at least 1.

2. Compounds according to Claim 1, characterized in that the rings of the unsaturated groups Z of formula (II) are selected from pyrrole, imidazole, pyrazole, oxazole, thiazole and triazole rings.

3. Compounds according to Claim 1, .characterized in that the rings of the unsaturated groups Z of formula (III) are selected from pyridine, pyrimidine, pyrazine, oxazine and triazine rings.

4. Compounds according to any one of the preceding claims, characterized in that two of the radicals R₇, R₈ and R₉ form a pyrrolidine ring, a piperidine ring, a piperazine ring or a morpholine ring, it being possible for the said ring to be unsubstituted or substituted by a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a tri(C₁-C₆ alkyl)silyl-C₁-C₆ alkyl radical, an amido radical, an aldehydo radical, a carboxyl radical, a C₁-C₆ alkylcarbonyl radical, a thio radical, a C1-C6 thioalkyl radical, a (C₁-C₆ alkyl)thio radical, an amino radical, or an amino radical protected by a (C₁-C₆ alkyl)carbonyl, carbamyl or (C₁-C₆ alkyl)sulphonyl radical.

5. Compounds according to any one of the preceding claims, characterized in that X⁻ is selected from a halogen atom, a hydroxide, a hydrogen sulphate or a C₁-C₆ alkyl sulphate.

6. Compounds according to any one of the preceding claims, characterized in that they are selected from:
- 3-(4-hydroxy-1-methyl-1H-indol-5-ylmethyl)-1-methylpyridinium methosulphate;
- 4-(4-hydroxy-1-methyl-1H-indol-5-ylmethyl)-1-methylpyridinium methosulphate;
- 3-[3-(4-hydroxy-5-(1-methylpyridinium)-4-ylmethylindol-1-yl)propyl]-1-methylimidazol-1-ium dimethosulphate;
- 4-(4-hydroxy-1-(2-hydroxyethyl)-1H-indol-5-ylmethyl)-1-methylpyridinium methosulphate;
- 3-[3-(4-hydroxy-5-(1-methylpyridinium)-5-ylmethyl-indol-1-yl)propyl]-1-methylimidazol-1-ium dimethosulphate;
- 3-[4-hydroxy-5-(1-methylpyridinium)-3-ylmethylindol-1-ylmethyl]-1-methylpyridinium dimethosulphate;
- 3-[3-(5-benzyl-4-hydroxyindol-1-yl)propyl]-1-methyl-3H-imidazol-1-ium methosulphate;
- [2-(4-hydroxy-1H-indol-3-yl)ethyl]trimethylammonium methosulphate;
- [2-(4-hydroxy-1-methyl-1H-indol-3-yl)ethyl]trimethylammonium methosulphate;
- (4-hydroxy-1-methyl-1H-indol-3-ylmethyl)trimethylammonium methosulphate;
- (4-hydroxy-1H-indol-3-ylmethyl)trimethylammonium methosulphate;
- {3-[(4-hydroxy-1H-indole-2-carbonyl)amino]propyl)trimethylammonium methosulphate;
- {3-[(4-hydroxy-1-methyl-1H-indole-2-carbonyl)amino]propyl}trimethylammonium methosulphate;
- {3-[(4-hydroxy-5-methyl-1H-indole-2-carbonyl)amino]propyl}trimethylammonium methosulphate;
- {3-[(4-hydroxy-1,5-dimethyl-1H-indole-2-carbonyl)amino]propyl}trimethylammonium methosulphate;
- 3-{3-[(4-hydroxy-1H-indole-2-carbonyl)amino]propyl}-1-methyl-3H-imidazol-1-ium methosulphate;
- 3-{3-[(4-hydroxy-1-methyl-1H-indole-2-carbonyl)amino]propyl}-1-methyl-3H-imidazol-1-ium methosulphate;
- 3-{3-[(4-hydroxy-5-methyl-1H-indole-2-carbonyl)amino]propyl}-1-methyl-3H-imidazol-1-ium methosulphate;
- 3-{3-[(4-hydroxy-l,5-dimethyl-1H-indole-2-carbonyl)amino]propyl}-1-methyl-3H-imidazol-1-ium methosulphate;
- {3-[(4-hydroxy-1H-indole-6-carbonyl)amino]propyl)trimethylammonium monochloride;
- {3-[(4-hydroxy-1-methyl-1H-indole-6-carbonyl)amino]propyl}trimethylammonium monochloride;
and their addition salts with an acid.

7. Compounds according to any one of the preceding claims, characterized in that the addition salts with an acid are selected from hydrochlorides, hydrobromides, sulphates, citrates, succinates, tartrates, lactates and acetates.

8. Use of the compounds of formula (I) as defined in any one of Claims 1 to 7 as a coupler for the oxidation dyeing of keratinous fibres and, in particular, of human keratinous fibres such as the hair.

9. Composition for the oxidation dyeing of keratinous fibres and, in particular, of human keratinous fibres such as the hair, characterized in that it comprises, in a medium appropriate for dyeing, at least one compound of formula (I) as defined in any one of Claims 1 to 7.

10. Composition according to Claim 9, characterized in that the compound or compounds of formula (I) represents or represent from 0.0005 to 12% by weight of the total weight of the dyeing composition.

11. Composition according to Claim 9, characterized in that the compound or compounds of formula (I) represents or represent from 0.005 to 6% by weight of the total weight of the dyeing composition.

12. Composition according to any one of Claims 9 to 11, characterized in that it includes one or more oxidation bases selected from para-phenylenediamines, bisphenylalkylenediamines, para-aminophenols, ortho-aminophenols and heterocyclic bases.

13. Composition according to Claim 12, characterized in that the para-phenylenediamines are selected from para-phenylenediamine, para-tolylene-diamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl) -para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine and their addition salts with an acid.

14. Composition according to Claim 12, characterized in that the bisphenylalkylenediamines are selected from N,N'-bis (β-hydroxyethyl) -N,N' -bis (4'-aminophenyl) -1, 3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)- tetramethylenediamine, N,N'-bis(ethyl)-N,N'-bis(4'-amino-3'-methylphenyl)ethylenediamine, 1, 8-bis(2,5-diaminophenoxy)-3,5-dioxaoctane, and their addition salts with an acid.

15. Composition according to Claim 12, characterized in that the para-aminophenols are selected from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2(β-hydroxyethylamino-methyl)phenol, 4-amino-2-fluorophenol, and their addition salts with an acid.

16. Composition according to Claim 12, characterized in that the ortho-aminophenols are selected from 2-aminophenol, 2-amino-5-methylphenol, 2-amino-6-methylphenol, 5-acetamido-2-aminophenol, and their addition salts with an acid.

17. Composition according to Claim 12, characterized in that the heterocyclic bases are selected from pyridine derivatives, pyrimidine derivatives and pyrazole derivatives.

18. Composition according to any one of Claims 12 to 17, characterized in that the oxidation base or bases represents or represent from 0.0005 to 12% by weight of the total weight of the dyeing composition.

19. Composition according to Claim 18, characterized in that the oxidation base or bases represents or represent from 0.005 to 6% by weight of the total weight of the dyeing composition.

20. Composition according to any one of Claims 9 to 19, characterized in that it includes one or more couplers selected from meta-phenylenediamines, meta-aminophenols, meta-diphenols and heterocyclic couplers.

21. Composition according to Claim 20, characterized in that the couplers are selected from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phenyl-3-methylpyrazol-5-one, and their addition salts with an acid.

22. Composition according to Claim 20 or 21, characterized in that the coupler or couplers represents or represent from 0.0001 to 10% by weight, approximately, of the total weight of the dyeing composition.

23. Composition according to Claim 22, characterized in that the coupler or couplers represents or represent from 0.005 to 5% by weight, approximately, of the total weight of the dyeing composition.

24. Composition according to any one of Claims 9 to 23, characterized in that the addition salts with an acid are selected from hydrochlorides, hydrobromides, sulphates, citrates, succinates, tartrates, lactates and acetates.

25. Method of dyeing keratinous fibres and, in particular, human keratinous fibres such as the hair, characterized in that at least one dyeing composition as defined in any one of Claims 9 to 24 is applied to these fibres and in that the colour is revealed at an acidic, neutral or alkaline pH with the aid of an oxidizing agent which is added to the dyeing composition right at the time of use or which is present in an oxidizing composition which is applied simultaneously or sequentially and separately.

26. Method according to Claim 25, characterized in that the oxidizing agent is selected from hydrogen peroxide, urea peroxide, alkali metal bromates, persalts such as perborates and persulphates, and enzymes such as peroxidases, laccases and oxidoreductases having 2 electrons.

27. Multi-compartment device, or multi-compartment dyeing kit, of which a first compartment contains a dyeing composition as defined in any one of Claims 9 to 24 and a second compartment contains an oxidizing composition.

## Patentansprüche

1. Verbindungen der folgenden Formel (I) und ihre Additionssalze mit einer Säure: 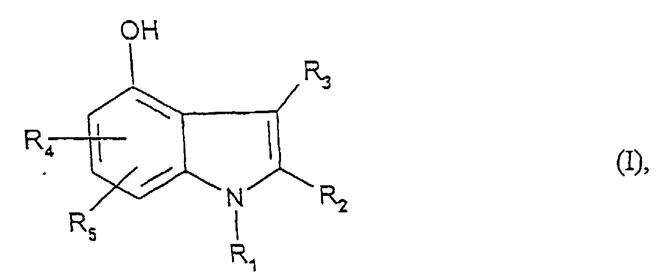 worin bedeuten:
- die Gruppe R₁ Wasserstoff; eine Gruppe Z; eine C₁₋₄-Alkylgruppe; eine C₁₋₄-Monohydroxyalkylgruppe; eine C₂₋₄-Polyhydroxyalkylgruppe; eine C₁₋₄-Alkoxy-C₁₋₄-alkylgrappe; eine C₁₋₄-Hydroxyalkoxy-C₁₋₄-alkylgruppe; eine C₁₋₄-Aminoalkylgruppe; eine C₁₋₄-Aminoalkylgruppe, deren Amin mit C₁₋₄-Alkyl, Acetyl, C₁₋₄-Monohydroxyalkyl oder C₂₋₄-Polyhydroxyalkyl mono- oder disubstituiert ist; eine C₁₋₄-Alkyl-C₁₋₄-thioalkylgruppe; eine C₁₋₄-Monohydroxyalkyl-C₁₋₄-thioalkylgruppe; eine C₂₋₄-Polyhydroxyalkyl-C₁₋₄-thioalkylgruppe; eine C₁₋₄-Carboxyalkylgruppe; eine C₁₋₄-Alkoxy-C₁₋₄-carbonylalkylgruppe; eine C₁₋₄-Acetylaminoalkylgruppe; eine C₁₋₄-Cyanoalkylgruppe; eine C₁₋₄-Trifluoralkylgruppe; eine C₁₋₄-Halogenalkylgruppe; eine C₁₋₄-Phosphoalkylgruppe oder eine C₁₋₄-Sulfoalkylgruppe;
- die Gruppen R₂ und R₃, die identisch oder voneinander verschieden sind, Wasserstoff; Halogen; eine Gruppe Z; eine Gruppe -NH-Z; eine C₁₋₄-Alkylgruppe; eine Carboxygruppe; eine C₁₋₄-Alkoxycarbonylgruppe oder eine Formylgruppe;
- die Gruppen R₄ und R₅, die identisch oder voneinander verschieden sind, Wasserstoff; Halogen; eine Gruppe Z; eine Gruppe -NH-Z; eine C₁₋₄-Alkylgruppe; eine C₁₋₄-Alkoxygruppe; eine Acetylaminogruppe; eine C₁₋₅-Monohydroxyalkylgruppe; eine C₂₋₄-Polyhydroxyalkylgruppe; eine C₁₋₄-Alkoxy-C₁₋₄-alkylgruppe; eine Thiophengruppe; eine Furangruppe; eine Phenylgruppe; eine C₁₋₄-Aralkylgruppe; eine Phenyl- oder C₁₋₄-Aralkylgruppe, die mit Halogen, C₁₋₄-Alkyl, Trifluormethyl, C₁₋₄-Alkoxy, Amino oder einer Aminogruppe substituiert ist, deren Amin mit C₁₋₄-Alkyl mono- oder disubstituiert ist; eine C₁₋₄-Alkyl-C₁₋₄-aminoalkylgruppe oder eine C₁₋₄-Dialkyl-C₁₋₄-aminoalkylgruppe;
- Z ist unter den ungesättigten kationischen Gruppen der folgenden Formeln (II) und (III) und den gesättigten kationischen Gruppen der folgenden Formel (IV) ausgewählt: 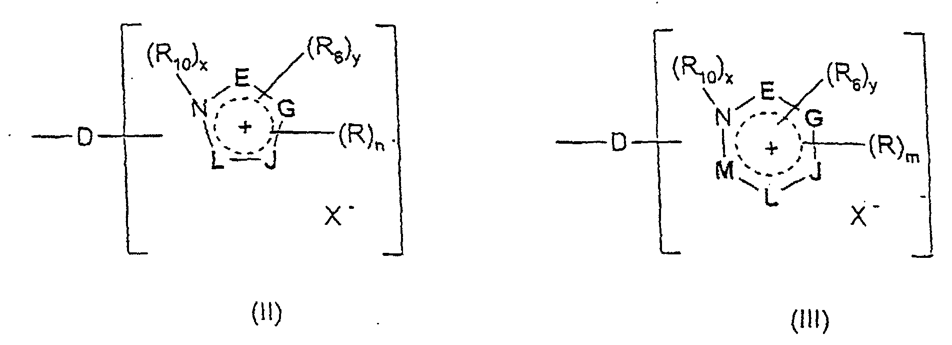 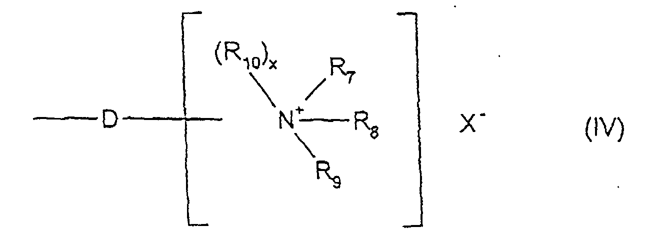 worin bedeuten:
- D eine Verbindungsgruppe, die eine geradkettige oder verzweigte Alkylgruppe mit vorzugsweise 1 bis 14 Kohlenstoffatomen bedeutet, die durch ein oder mehrer Heteroatome unterbrochen sein kann, beispielsweise Sauerstoff, Schwefel oder Stickstoff, und die mit einer oder mehreren Hydroxygruppen oder C₁₋₆-Alkoxygruppen substituiert sein kann und eine oder mehrere Ketongruppen tragen kann;
- die Atome E, G, J, L und M, die identisch oder voneinander verschieden sind, Kohlenstoff, Sauerstoff, Schwefel oder Stickstoff;
- n Null oder eine ganze Zahl von 1 bis 4;
- m Null oder eine ganze Zahl von 1 bis 5;
- die Gruppen R, die identisch oder voneinander verschieden sind, Z; Halogen; Hydroxy; C₁₋₆-Alkyl; C₁₋₆-Monohydroxyalkyl; C₂₋₆-Polyhydroxyalkyl; Nitro; Cyano; C₁₋₆-Cyanoalkyl; C₁₋₆-Alkoxy; C₁₋₆-Trialkyl-C₁₋₆-silanalkyl; Amido; Aldehydo; Carboxy; C₁₋₆-Alkylcarbonyl; Thio; C₁₋₆-Thioalkyl; C₁₋₆-Alkylthio; Amino; eine Aminogruppe, die mit C₁₋₆-Alkylcarbonyl, Carbamoyl oder C₁₋₆-Alkylsulfonyl geschützt ist; oder eine Gruppe NHR" oder NR"R"', worin die Gruppen R" und R"', die identisch oder voneinander verschieden sind, C₁₋₆-Alkyl, C₁₋₆-Monohydroxyalkyl oder C₂₋₆-Polyhydroxyalkyl bedeuten;
- R₆ C₁₋₆-Alkyl; C₁₋₆-Monohydroxyalkyl; C₂₋₆-Polyhydroxyalkyl; C₁₋₆-Cyanoalkyl; C₁₋₆-Trialkyl-C₁₋₆-silanalkyl; C₁₋₆-Alkoxy-C₁₋₆-alkyl; C₁₋₆-Carbamoylalkyl; C₁₋₆-Alkyl-C₁₋₆-carboxyalkyl; Benzyl; oder eine Gruppe Z der oben definierten Formel (II), (III) oder (IV);
- R₇, R₈ und R₉, die identisch oder voneinander verschieden sind, C₁₋₆-Alkyl; C₁₋₆-Monohydroxyalkyl; C₂₋₆-Polyhydroxyalkyl; C₁₋₆-Alkoxy-C₁₋₆-alkyl; C₁₋₆-Cyanoalkyl; Aryl; Benzyl; C₁₋₆-Amidoalkyl; C₁₋₆-Trialkyl-C₁₋₆-silanalkyl; oder eine C₁₋₆-Aminoalkylgruppe, deren Amin mit C₁₋₆-Alkylcarbonyl, Carbamoyl oder C₁₋₆-Alkylsulfonyl geschützt ist; wobei zwei der Gruppen R₇, R₈ und R₉ auch gemeinsam mit dem Stickstoffatom, an das sie gebunden sind, einen 5- oder 6-gliedrigen gesättigten Ring oder einen Ring bilden können, der ein oder mehrere Heteroatome enthält, wobei der Ring unsubstituiert vorliegen oder mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, C₁₋₆-Trialkyl-C₁₋₆-silanalkyl, Amido, Aldehydo, Carboxy, C₁₋₆-Ketoalkyl, Thio, C₁₋₆-Thioalkyl, C₁₋₆-Alkylthio, Amino, oder einer Aminogruppe, die mit C₁₋₆-Alkylcarbonyl, Carbamoyl oder C₁₋₆-Alkylsulfonyl geschützt ist, substituiert sein kann;
wobei eine der Gruppen R₇, R₈ und R₉ auch eine zweite Gruppe Z bedeuten kann, die identisch mit der ersten Gruppe Z oder von ihr verschieden ist;
- R₁₀ C₁₋₆-Alkyl; C₁₋₆-Monohydroxyalkyl; C₂₋₆-Polyhydroxyalkyl; Aryl; Benzyl; C₁₋₆-Aminoalkyl; eine C₁₋₆-Aminoalkylgruppe, deren Amin mit C₁₋₆-Alkylcarbonyl, Carbamoyl oder C₁₋₆-Alkylsulfonyl geschützt ist; C₁₋₆-Carboxyalkyl; C₁₋₆-Cyanoalkyl; C₁₋₆-Carbamoylalkyl; C₁₋₆-Trifluoralkyl; C₁₋₆-Trialkyl-C₁₋₆-silanalkyl; C₁₋₆-Sulfonamidoalkyl; C₁₋₆-Alkyl-C₁₋₆-carboxyalkyl; C₁₋₆-Alkyl-C₁₋₆-sulfinylalkyl; C₁₋₆-Alyl-C₁₋₆-sulfonylalkyl; C₁₋₆-Alkyl-C₁₋₆-ketoalkyl; N-C₁₋₆-Alkylcarbamoylalkyl; N-C₁₋₆-Alkyl-C₁₋₆-sulfonamidoalkyl;
- x und y 0 oder 1;
- mit den folgenden Bedingungen:
- in den kationischen ungesättigten Gruppen der Formel (II):
- wenn x = 0, ist die Verbindungsgruppe D an das Stickstoffatom gebunden,
- wenn x = 1, ist die Verbindungsgruppe D an E, G, J oder L gebunden,
- y kann nur den Wert 1 annehmen, wenn
1) E, G, J oder L gleichzeitig ein Kohlenstoffatom bedeuten und die Gruppe R₆ von dem Stickstoffatom des ungesättigten Rings getragen wird,
2) mindestens eines der Atome E, G, J oder L Stickstoff bedeutet, an den die Gruppe R₆ gebunden ist;
- in den kationischen ungesättigten Gruppen der Formel (III):
- wenn x = 0, ist die Verbindungsgruppe D an das Stickstoffatom gebunden,
- wenn x = 1, ist die Verbindungsgruppe D an E, G, J, L oder M gebunden,
- y kann nur den Wert 1 annehmen, wenn mindestens eines der Atome E, G, J, L oder M ein zweiwertiges Atom bedeutet und die Gruppe R₆ von dem Stickstoffatom des ungesättigten Rings getragen wird,
- in den kationischen Gruppen der Formel (IV):
- wenn x = 0, ist die Verbindungsgruppe an ein Stickstoffatom der Gruppen R₇ bis R₉ gebunden,
- wenn x = 1, bilden zwei der Gruppen R₇ bis R₉ gemeinsam mit dem Stickstoffatom, an das sie gebunden sind, einen oben definierten 5- oder 6-gliedrigen Ring und die Verbindungsgruppe D ist an ein Kohlenstoffatom des gesättigten Rings gebunden;
- X⁻ ein einwertiges oder zweiwertiges Anion;
- mit der Maßgabe, daß die Anzahl der kationischen Gruppen Z der Formel (II), (III) oder (IV) mindestens 1 ist.

2. Verbindungen nach Anspruch 1, dadurch gekennzeichnet, daß die Ringe der ungesättigten Gruppen Z der Formel (II) unter Pyrrol, Imidazol, Pyrazol, Oxazol, Thiazol und Triazol ausgewählt sind.

3. Verbindungen nach Anspruch 1, dadurch gekennzeichnet, daß die Ringe der ungesättigten Gruppen Z der Formel (III) unter Pyridin, Pyrimidin, Pyrazin, Oxazin und Triazin ausgewählt sind.

4. Verbindungen nach einem der vorhergehenden Ansprüche, dadurch gekennzeichnet, daß die Gruppen R₇, R₈ und R₉ einen Pyrrolidin-, Piperidin-, Piperazin- oder Morpholinring bilden, wobei der Ring unsubstituiert vorliegen oder mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, C₁₋₆-Trialkyl-C₁₋₆-silanalkyl, Amido, Aldehydo, Carboxy, C₁₋₆-Alkylcarbonyl, Thio, C₁₋₆-Thioalkyl, C₁₋₆-Alkylthio, Amino, oder einer Aminogruppe, die mit C₁₋₆-Alkylcarbonyl, Carbamoyl oder C₁₋₆-Alkylsulfonyl geschützt ist, substituiert sein kann.

5. Verbindungen nach einem der vorhergehenden Ansprüche, dadurch gekennzeichnet, daß X⁻ unter Halogen, Hydroxid, Hydrogensulfat oder C₁₋₆-Alkylsulfat ausgewählt ist.

6. Verbindungen nach einem der vorhergehenden Ansprüche, dadurch gekennzeichnet, daß sie ausgewählt sind unter:
- 3-(4-Hydroxy-1-methyl-1H-indol-5-ylmethyl)-1-methyl-pyridinium-methosulfat;
- 4-(4-Hydroxy-1-methyl-1H-indol-5-ylmethyl)-1-methyl-pyridinium-methosulfat;
- 3-[3-(4-Hydroxy-5-(1-methylpyridinium)-4-ylmethyl-indol-1-yl)-propyl]-1-methyl-imidazol-1-ium-dimethosulfat;
- 4-(4-Hydroxy-1-(2-hydroxyethyl)-1H-indol-5-ylmethyl)-1-methyl-pyridiniummethosulfat;
- 3-[3-(4-Hydroxy-5-(1-methylpyridinium)-5-ylmethyl-indol-1-yl)-propyl]-1-methyl-imidazol-1-ium-dimethosulfat;
- 3-[4-Hydroxy-5-(1-methylpyridinium)-3-ylmethyl-indol-1-ylmethyl]-1-methylpyridinium-dimethosulfat;
- 3-[3-(5-Benzyl-4-hydroxy-indol-1-yl)-propyl]-1-methyl-3H-imidazol-1-ium-methosulfat;
- [2-(4-Hydroxy-1H-indol-3-yl)-ethyl]-trimethyl-ammonium-methosulfat;
- [2-(4-Hydroxy-1-methyl-1H-indol-3-yl)-ethyl]-trimethyl-ammonium-methosulfat;
- (4-Hydroxy-1-methyl-1H-indol-3-ylmethyl]-trimethyl-ammonium-methosulfat;
- (4-Hydroxy-1H-indol-3-ylmethyl]-trimethyl-ammonium-methosulfat;
- {3-[(4-Hydroxy-1H-indol-2-carbonyl)-amino]-propyl}-trimethyl-ammonium-methosulfat;
- {3-[(4-Hydroxy-1-methyl-1H-indol-2-carbonyl)-amino]-propyl}-trimethyl-ammonium-methosulfat;
- {3-[(4-Hydroxy-5-methyl-1H-indol-2-carbonyl)-amino]-propyl}-trimethyl-ammonium-methosulfat;
- {3-[(4-Hydroxy-1,5-dimethyl-1H-indol-2-carbonyl)-amino]-propyl}-trimethyl-ammonium-methosulfat;
- 3-{3-[{4-Hydroxy-1H-indol-2-carbonyl)-amino]-propyl}-1-methyl-3H-imidazol-1-ium-methosulfat;
- 3-{3-[{4-Hydroxy-1-methyl-1H-indol-2-carbonyl)-amino]-propyl}-1-methyl-3H-imidazol-1-ium-methosulfat;
- 3-{3-[{4-Hydroxy-5-methyl-1H-indol-2-carbonyl)-amino]-propyl]-1-methyl-3H-imidazol-1-ium-methosulfat;
- 3-{3-[{4-Hydroxy-1,5-dimethyl-1H-indol-2-carbonyl)-amino]-propyl)-1-methyl-3H-imidazol-1-ium-methosulfat;
- {3-[(4-Hydroxy-1H-indol-6-carbonyl)-amino]-propyl}-trimethyl-ammonium-monochlorid;
- {3-[(4-Hydroxy-1-methyl-1H-indol-6-carbonyl)-amino]-propyl)-trimethyl-ammonium-monochlorid; und
- deren Additionssalze mit einer Säure.

7. Verbindungen nach einem der vorhergehenden Ansprüche, dadurch gekennzeichnet, daß die Additionssalze mit einer Säure unter den Hydrochloriden, Hydrobromiden, Sulfaten, Citraten, Succinaten, Tartraten, Lactaten und Acetaten ausgewählt sind.

8. Verwendung der Verbindungen der Formel (I) nach einem der Ansprüche 1 bis 7 als Kuppler zum oxidativen Färben von Keratinfasern und insbesondere menschlichen Keratinfasern, wie dem Haar.

9. Zusammensetzung zum oxidativen Färben von Keratinfasern und insbesondere menschlichen Keratinfasern, wie dem Haar, dadurch gekennzeichnet, daß sie in einem zum Färben geeigneten Medium mindestens eine Verbindung der Formel (I) nach einem der Ansprüche 1 bis 7 enthält.

10. Zusammensetzung nach Anspruch 9, dadurch gekennzeichnet, daß die Verbindung(en) der Formel (I) 0,0005 bis 12 Gew.-% des Gesamtgewichts der Färbemittelzusammensetzung ausmachen.

11. Zusammensetzung nach Anspruch 9, dadurch gekennzeichnet, daß die Verbindung(en) der Formel (I) 0,005 bis 6 Gew.-% des Gesamtgewichts der Färbemittelzusammensetzung ausmachen.

12. Zusammensetzung nach einem der Ansprüche 9 bis 11, dadurch gekennzeichnet, daß sie eine oder mehrere Oxidationsbasen enthält, die unter den p-Phenylendiaminen, Bisphenylalkylendiaminen, p-Aminophenolen, o-Amino-phenolen und heterocyclischen Basen ausgewählt sind.

13. Zusammensetzung nach Anspruch 12, dadurch gekennzeichnet, daß die p-Phenylendiamine unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anhin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, N-β-Hydroxyethyloxy-p-phenylendiamin, 2-β-Acetylaminoethyloxy-p-phenylendiamin, N-(β-Methoxyethyl)-p-phenylendiamin und deren Additionssalze mit einer Säure ausgewählt sind.

14. Zusammensetzung nach Anspruch 12, dadurch gekennzeichnet, daß die Bisphenylalkylendiamine unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin, 1,8-Bis(2,5-diaminophenoxy)-3,5-dioxaoctan und deren Additionssalze mit einer Säure ausgewählt sind.

15. Zusammensetzung nach Anspruch 12, dadurch gekennzeichnet, daß die p-Aminophenole unter p-Aminophenol, 4-Amino-3-methyl-phenol, 4-Amino-3-fluorphenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydxoxyethylaminomethyl)-phenol, 4-Amino-2-fluor-phenol und deren Additionssalze mit einer Säure ausgewählt sind

16. Zusammensetzung nach Anspruch 12, dadurch gekennzeichnet, daß die o-Aminophenole unter 2-Aminophenol, 2-Amino-5-methyl-phenol, 2-Amino-6-methyl-phenol, 5-Acetamido-2-amino-phenol und deren Additionssalzen mit einer Säure ausgewählt sind.

17. Zusammensetzung nach Anspruch 12, dadurch gekennzeichnet, daß die heterocyclischen Basen unter den Pyridinderivaten, Pyrimidinderivaten und Pyrazolderivaten ausgewählt sind.

18. Zusammensetzung nach einem der Ansprüche 12 bis 17, dadurch gekennzeichnet, daß die Oxidationsbase(n) 0,0005 bis 12 Gew.-% des Gesamtgewichts der Färbemittelzusammensetzung ausmachen.

19. Zusammensetzung nach Anspruch 18, dadurch gekennzeichnet, daß die Oxidations-base(n) 0,005 bis 6 Gew.-% des Gesamtgewichts der Färbemittelzusammensetzung ausmachen.

20. Zusammensetzung nach einem der Ansprüche 9 bis 19, dadurch gekennzeichnet, daß einen oder mehrere Kuppler enthält, der unter den m-Phenylendiaminen, m-Aminophenolen, m-Dihydroxybenzolen und den heterocyclischen Kupplern ausgewählt sind.

21. Zusammensetzung nach Anspruch 20, dadurch gekennzeichnet, daß die Kuppler unter 2-Methyl-5-amino-phenol, 5-N-(β-Hydroxyethyl)amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diamino-Benzol, 1,3-Bis(2,4-diaminophenoxy)-propan, Sesamol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen ausgewählt sind.

22. Zusammensetzung nach Anspruch 20 oder 21, dadurch gekennzeichnet, daß die Kuppler etwa 0,0001 bis 10 Gew.-% des Gesamtgewichts der Färbemittelzusammensetzung ausmachen.

23. Zusammensetzung nach Anspruch 22, dadurch gekennzeichnet, daß die Kuppler etwa 0,005 bis 5 Gew.-% des Gesamtgewichts der Färbemittelzusammensetzung ausmachen.

24. Zusammensetzung nach einem der Ansprüche 9 bis 23, dadurch gekennzeichnet, daß die Additionssalze mit einer Säure unter den Hydrochloriden, Hydrobromiden Sulfaten, Citraten, Succinaten, Tartraten, Lactaten und Acetaten ausgewählt sind.

25. Verfahren zum Färben von Keratinfasern und insbesondere menschlichen Keratinfasern, wie dem Haar, dadurch gekennzeichnet, daß auf die Fasern mindestens eine Färbemittelzusammensetzung nach einem der Ansprüche 9 bis 24 aufgetragen wird und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

26. Verfahren nach Anspruch 25, dadurch gekennzeichnet, daß das Oxidationsmittel unter Wasserstoffperoxid, Harnstoffperoxid, Alkalimetallbromaten, Salzen von Persäuren, wie Perboraten und Persulfaten, und Enzymen, wie Peroxydasen, Laccasen und Oxydoreductasen mit 2 Elektronen, ausgewählt ist.

27. Vorrichtung mit mehreren Abteilungen oder "Kit" zum Färben mit mehreren Abteilungen, wobei eine erste Abteilung eine Färbemittelzusammensetzung nach einem der Ansprüche 9 bis 24 und eine zweite Abteilung eine oxidierende Zusammensetzung enthält.
